# EUROPEAN PATENT SPECIFICATION

(11) **EP 3 664 878 B1**
(45) Date of publication and mention of the grant of the patent: **02.10.2024**
(21) Application number: 18765732.5
(22) Date of filing: 08.08.2018
(51) Int. Cl.: A61M 15/06, A24F 47/00, A61M 15/00, A24F 42/20, A24F 42/60

(54) **VAPOR INSERT**
DAMPFEINSATZ
INSERT POUR VAPEUR

(30) Priority: 11.08.2017 US 201762544163 P
(43) Date of publication of application: 17.06.2020
(73) Proprietor: Philip Morris Products S.A., 2000 Neuchâtel (CH)
(72) Inventor: STENZLER, Alex, Long Beach California 90804 (US); HAN, Steve, Huntington Beach California 92648 (US); TIBBATTS, James, Cambridge CB23 7PY (GB); ELLIS, Steven, Oro-Medonte Ontario L0L 2E0 (CA)
(74) Representative: Reddie & Grose LLP
(86) International application number: PCT/IB2018/055980
(87) International publication number: WO 2019/030697

(56) References cited:
- WO-A1-2016/168276
- WO-A1-2017/108394
- CN-Y- 2 474 747
- GB-A- 2 313 311
- US-A- 3 789 855
- US-A- 4 358 371
- US-A1- 2015 313 278
- US-A1- 2017 006 917
- US-A1- 2017 049 153

## Description

### BACKGROUND OF THE INVENTION

There are a variety of devices on the market for inhaled media delivery, including gas inhalers, dry powder inhalers, and aerosol inhalers. The media inhaler devices can be utilized for medicament delivery and/or recreational use, but often do not provide the user with the ability to modify the inhalation experience outside of exchanging the inhaled media.

US 2017/0049153 A1 discloses an atomizer for use with vaporizers and electronic cigarettes, the atomizer comprising an inhaler having a first end and a second end through both of which air flow passage partially extends; a chamber coupled to said second end of said inhaler; a heating element positioned within the chamber to be in contact with a liquid. In an embodiment, the atomizer includes a housing and has openings communicating the inner and outer of the housing.

There is a need in the art for a means of enhancing inhaled media. The present invention addresses this need.

### SUMMARY OF THE INVENTION

In one aspect, the invention relates to a vapor insert device comprising: a hollow cylinder having a porous surface, an open anterior end, and an open posterior end; an anterior flange attached to the open anterior end; a posterior flange attached to the open posterior end; and one or more struts extending from the anterior flange to the posterior flange; the anterior flange and the posterior flange each has a diameter that is larger than a diameter of the hollow cylinder to form a circumferential space around the hollow cylinder.

In one embodiment, the porous surface is constructed from a wire mesh. In one embodiment, the porous surface is constructed from a woven textile. In one embodiment, the porous surface is constructed from a solid material having one or more holes or slits.

The device further comprises one or more struts extending from the anterior flange to the posterior flange. In one embodiment, the one or more struts partition the circumferential space by further extending from the surface of the hollow cylinder to the outer edge of the anterior flange and the posterior flange.

In one embodiment, the posterior flange comprises one or more apertures. In one embodiment, the anterior flange, the posterior flange, or both are at least partially constructed from a rubber or silicon material.

In one embodiment, the circumferential space fits one or more vapor sources. In one embodiment, the vapor source is selected from the group consisting of tobacco leaves, gel beads, flavoring agents, tea leaves, coffee grounds, and infused fibrous pads.

### BRIEF DESCRIPTION OF THE DRAWINGS

The following detailed description of embodiments of the invention will be better understood when read in conjunction with the appended drawings. It should be understood, however, that the invention is not limited to the precise arrangements and instrumentalities of the embodiments shown in the drawings.
Figure 1A and Figure 1B depict perspective and side views of an exemplary vapor insert.
Figure 2A and Figure 2B depict perspective front and rear views of an exemplary vapor insert having apertures.
Figure 3A and Figure 3B depict an exemplary vapor insert placed within the airway of a media inhaler.

### DETAILED DESCRIPTION

The present invention provides devices for holding one or more vapor sources in a media inhaler. The devices permit vapor from the one or more vapor sources to be introduced and entrained into a stream of inhaled media.

### Definitions

It is to be understood that the figures and descriptions of the present invention have been simplified to illustrate elements that are relevant for a clear understanding of the present invention, while eliminating, for the purpose of clarity, many other elements typically found in the art. Those of ordinary skill in the art may recognize that other elements and/or steps are desirable and/or required in implementing the present invention. However, because such elements and steps are well known in the art, and because they do not facilitate a better understanding of the present invention, a discussion of such elements and steps is not provided herein. The disclosure herein is directed to all such variations and modifications to such elements and methods known to those skilled in the art.

Unless defined elsewhere, all technical and scientific terms used herein have the same meaning as commonly understood by one of ordinary skill in the art to which this invention belongs. Although any methods and materials similar or equivalent to those described herein can be used in the practice or testing of the present invention, exemplary methods and materials are described.

As used herein, each of the following terms has the meaning associated with it in this section.

The articles "a" and "an" are used herein to refer to one or to more than one (i.e., to at least one) of the grammatical object of the article. By way of example, "an element" means one element or more than one element.

"About" as used herein when referring to a measurable value such as an amount, a temporal duration, and the like, is meant to encompass variations of ±20%, ±10%, ±5%, ±1%, and ±0.1% from the specified value, as such variations are appropriate.

Throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6, etc., as well as individual numbers within that range, for example, 1, 2, 2.7, 3, 4, 5, 5.3, 6, and any whole and partial increments therebetween. This applies regardless of the breadth of the range.

### Vapor Insert

The present invention provides vapor insert devices that are sized to fit within the airway of a media inhaler. The vapor inserts are capable of holding one or more vapor sources within the airway of the media inhaler. The vapor inserts permit vapor from the one or more vapor sources to enter the airway of the media inhaler, whereupon inhalation by a user entrains vapor into the media stream to simultaneously deliver media and vapor to the user.

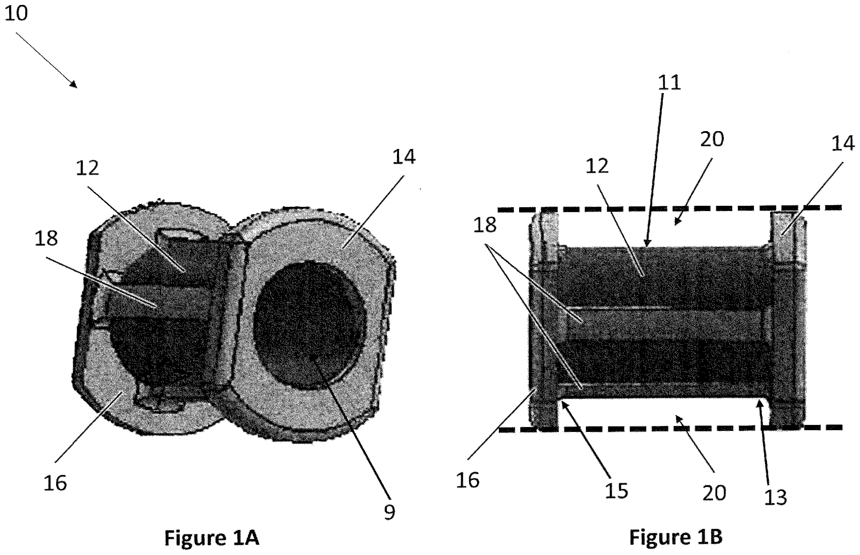

Referring now to Figure 1A and Figure 1B, an exemplary vapor insert 10 is depicted. Vapor insert 10 comprises cylinder 12, anterior flange 14, and posterior flange 16. Cylinder 12 comprises a hollow interior and is open at both cylinder ends. Cylinder 12 comprises a surface 11 that permits the passage of air and vapor. In some embodiments, cylinder 12 comprises a mesh surface 11 that passes air and vapor, such as a woven textile or wire mesh material. In other embodiments, cylinder 12 comprises a solid surface 11 having one or more holes or slits that pass air and vapor. Cylinder 12 can have any suitable length, such as a length that fits within the airway of a media inhaler. Cylinder 12 can have any suitable width, preferably a width that is smaller than the airway of a media inhaler.

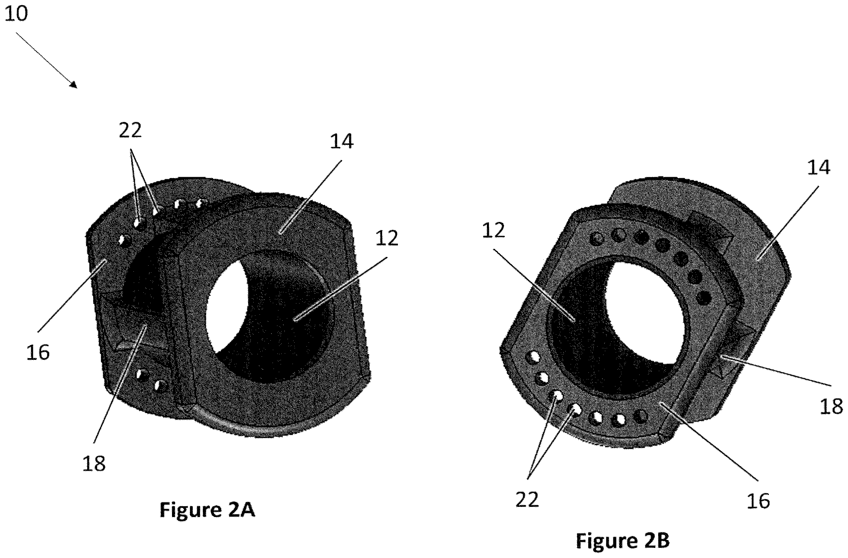

The anterior open end 13 of cylinder 12 is joined to anterior flange 14 and the posterior open end 15 of cylinder 12 is joined to posterior flange 16. Anterior flange 14 and posterior flange 16 are sized and shaped to fit flush within the airway of a media inhaler to securely position cylinder 12 within the airway. Referring now to Figure 1B, vapor insert 10 is depicted with dashed lines to simulate the lumen of a media inhaler airway. Anterior flange 14 and posterior flange 16 have larger diameters than cylinder 12, and the diameter difference forms circumferential space 20 around cylinder 12. Anterior flange 14 and posterior flange 16 can be constructed from any suitable material, such as a metal or plastic. In certain embodiments, anterior flange 14, posterior flange 16, or both can be at least partially constructed from a rubber or silicone material to enhance fit and grip within the airway of a media inhaler. In certain embodiments, posterior flange 16 comprises one or more apertures 22 (Figure 2A and Figure 2B).

One or more struts 18 extend between anterior flange 14 and posterior flange 16. The one or more struts 18 are constructed from a stiff material, such as a metal or plastic, and enhance the structural stiffness of vapor insert 10, particularly if cylinder 12 is constructed from a flexible material. In certain embodiments, the one or more struts 18 can be sized to also extend from the outer surface of cylinder 12 and the outer edge of anterior flange 14 and posterior flange 16 (not pictured). In this manner, the one or more struts 18 partition circumferential space 20 into two or more compartments.

The various components of the present invention described above can be made using any suitable method known in the art. The method of making may vary depending on the materials used. For example, components substantially comprising a metal may be milled from a larger block of metal or may be cast from molten metal. Likewise, components substantially comprising a plastic or polymer may be milled from a larger block, cast, or injection molded. In some embodiments, the devices may be made using 3D printing or other additive manufacturing techniques commonly used in the art.

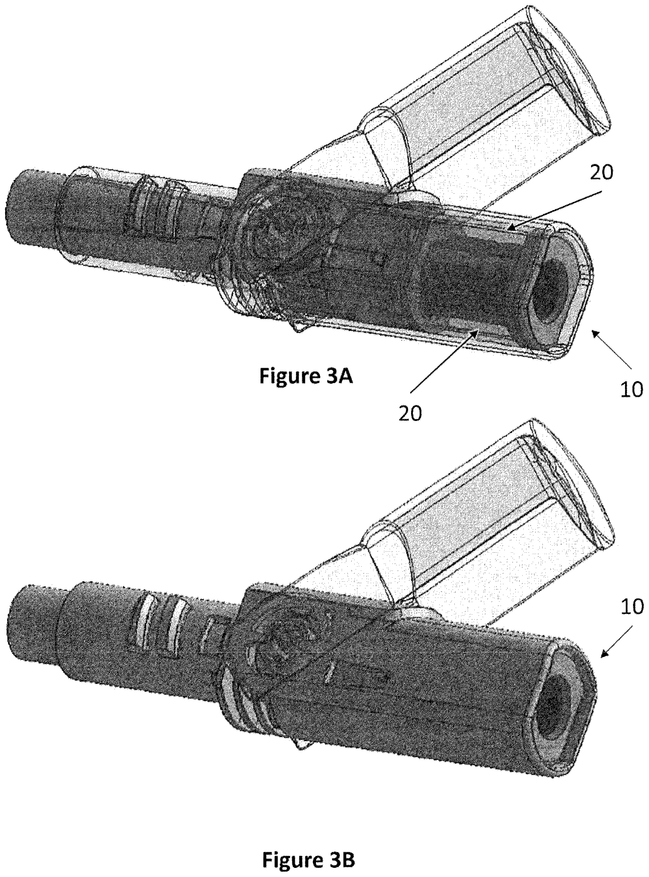

Referring now to Figure 3A and Figure 3B, an exemplary vapor insert 10 is depicted being inserted within the airway of a media inhaler. In the top image, the media inhaler is rendered semitransparent to depict circumferential space 20. In this manner, one or more vapor sources can be held within circumferential space 20. The one or more vapor sources can be any suitable aromatic material, such as gel beads, flavoring agents, tobacco leaves, tea leaves, coffee grounds, infused fibrous pads, and the like. As described elsewhere herein, cylinder 12 comprises a surface that permits the passage of air and vapor. When a user applies a vacuum to the media inhaler airway to draw in a dose of media, the flow of air passes through vapor insert 10. Air passing through vapor insert 10 may entrain vapor diffusing through cylinder 12 into its hollow interior. Air passing through vapor insert 10 may also pass through cylinder 12 to enter circumferential space 20 and entrain additional vapor. In certain embodiments, a vapor insert 10 having a posterior flange 16 with apertures 22 enhances the passing of air into circumferential space 20 to entrain additional vapor.

The invention further includes kits comprising one or more vapor inserts and one or more vapor sources. The one or more vapor sources can be provided as loose material or in small porous packets sized to fit within the circumferential space of the one or more vapor inserts for convenient insertion and removal.

## Claims

1. A vapor insert device (10) suitable for holding one or more vapor sources and sized to fit within the airway of a media inhaler, the vapor insert device comprising:
a hollow cylinder (12) having a porous surface, an anterior open end, and a posterior open end;
an anterior flange (14) joined to the anterior open end;
a posterior flange (16) joined to the posterior open end; and
the anterior flange (14) and the posterior flange (16) each has a diameter that is larger than a diameter of the hollow cylinder (12) to form a circumferential space (20) around the hollow cylinder (12), **characterised in that** the vapor insert device further comprises one or more struts (18) extending from the anterior flange to the posterior flange.

2. The device of claim 1, wherein the porous surface is constructed from a wire mesh.

3. The device of claim 1, wherein the porous surface is constructed from a woven textile.

4. The device of claim 1, wherein the porous surface is constructed from a solid material having one or more holes or slits.

5. The device of claim 1, wherein the one or more struts partition the circumferential space by further extending from the surface of the hollow cylinder to the outer edge of the anterior flange (14) and the posterior flange (16).

6. The device of claim 1, wherein the posterior flange (16) comprises one or more apertures (22).

7. The device of claim 1, wherein the anterior flange (14), the posterior flange (16), or both are at least partially constructed from a rubber or silicon material.

8. The device of claim 1, wherein the circumferential space (20) fits one or more vapor sources.

9. The device of claim 8, wherein the vapor source is selected from the group consisting of tobacco leaves, gel beads, flavoring agents, tea leaves, coffee grounds, and infused fibrous pads.

## Patentansprüche

1. Dampfeinsatzvorrichtung (10), geeignet zum Halten einer oder mehrerer Dampfquellen und so bemessen, dass sie in den Luftweg eines Medieninhalators passt, wobei die Dampfeinsatzvorrichtung umfasst:
einen Hohlzylinder (12), aufweisend eine poröse Fläche, ein vorderes offenes Ende und ein hinteres offenes Ende;
einen mit dem vorderen offenen Ende verbundenen vorderen Flansch (14);
einen mit dem hinteren offenen Ende verbundenen hinteren Flansch (16); und
wobei der vordere Flansch (14) und der hintere Flansch (16) jeweils einen Durchmesser aufweisen, der größer ist als ein Durchmesser des Hohlzylinders (12), um einen Umfangsraum (20) um den Hohlzylinder (12) zu bilden, **dadurch gekennzeichnet, dass** die Dampfeinsatzvorrichtung ferner eine oder mehrere Streben (18) aufweist, die sich von dem vorderen Flansch zu dem hinteren Flansch erstrecken.

2. Vorrichtung nach Anspruch 1, wobei die poröse Fläche aus einem Drahtgeflecht hergestellt ist.

3. Vorrichtung nach Anspruch 1, wobei die poröse Fläche aus einem gewebten Textil hergestellt ist.

4. Vorrichtung nach Anspruch 1, wobei die poröse Fläche aus einem festen Material hergestellt ist, das ein oder mehrere Löcher oder Schlitze aufweist.

5. Vorrichtung nach Anspruch 1, wobei die eine oder die mehreren Streben den Umfangsraum durch weiteres sich erstrecken von der Fläche des Hohlzylinders zu dem äußeren Rand des vorderen Flansches (14) und des hinteren Flansches (16) unterteilen.

6. Vorrichtung nach Anspruch 1, wobei der hintere Flansch (16) eine oder mehrere Öffnungen (22) aufweist.

7. Vorrichtung nach Anspruch 1, wobei der vordere Flansch (14), der hintere Flansch (16) oder beide wenigstens teilweise aus einem Gummi- oder Silikonmaterial hergestellt sind.

8. Vorrichtung nach Anspruch 1, wobei der Umfangsraum (20) eine oder mehrere Dampfquellen aufnimmt.

9. Vorrichtung nach Anspruch 8, wobei die Dampfquelle ausgewählt ist aus der Gruppe bestehend aus Tabakblättern, Gelkügelchen, Geschmacksstoffen, Teeblättern, Kaffeesatz und infundierten Faserkissen.

## Revendications

1. Dispositif d'insert de vapeur (10) approprié pour contenir une ou plusieurs sources de vapeur et dimensionné pour s'adapter au sein de la voie d'air d'un inhalateur de support, le dispositif d'insert de vapeur comprenant :
un cylindre creux (12) ayant une surface poreuse, une extrémité ouverte antérieure et une extrémité ouverte postérieure ;
une bride antérieure (14) jointe à l'extrémité ouverte antérieure ;
une bride postérieure (16) jointe à l'extrémité ouverte postérieure ; et
la bride antérieure (14) et la bride postérieure (16) ont chacune un diamètre qui est supérieur à un diamètre du cylindre creux (12) pour former un espace circonférentiel (20) autour du cylindre creux (12), **caractérisé en ce que** le dispositif d'insert de vapeur comprend en outre une ou plusieurs entretoises (18) s'étendant de la bride antérieure à la bride postérieure.

2. Dispositif selon la revendication 1, dans lequel la surface poreuse est construite à partir d'un treillis métallique.

3. Dispositif selon la revendication 1, dans lequel la surface poreuse est construite à partir d'un textile tissé.

4. Dispositif selon la revendication 1, dans lequel la surface poreuse est construite à partir d'un matériau solide ayant un(e) ou plusieurs trous ou fentes.

5. Dispositif selon la revendication 1, dans lequel les une ou plusieurs entretoises divisent l'espace circonférentiel par extension supplémentaire de la surface du cylindre creux jusqu'au bord extérieur de la bride antérieure (14) et de la bride postérieure (16).

6. Dispositif selon la revendication 1, dans lequel la bride postérieure (16) comprend un ou plusieurs orifices (22).

7. Dispositif selon la revendication 1, dans lequel la bride antérieure (14), la bride postérieure (16), ou les deux, sont au moins partiellement construites à partir d'un matériau en caoutchouc ou en silicone.

8. Dispositif selon la revendication 1, dans lequel l'espace circonférentiel (20) admet une ou plusieurs sources de vapeur.

9. Dispositif selon la revendication 8, dans lequel la source de vapeur est sélectionnée dans le groupe constitué par des feuilles de tabac, des billes de gel, des agents aromatisants, des feuilles de thé, du marc de café et des tampons fibreux infusés.
